# EUROPÄISCHE PATENTSCHRIFT

(11) **EP 3 522 834 B1**
(45) Veröffentlichungstag und Bekanntmachung des Hinweises auf die Patenterteilung: **07.12.2022**
(21) Anmeldenummer: 17780374.9
(22) Anmeldetag: 02.10.2017
(51) Int. Cl.: A61F 2/50

(54) **VORRICHTUNG ZUM ERSTELLEN EINES GIPSABDRUCKS EINES KÖRPERGLIEDSTUMPFS MIT VERBINDER**
DEVICE FOR FORMING A MOULD OF A STUMP COMPRISING A CONNECTOR
DISPOSITIF POUR FORMER UN MOULE D'UN MOIGNON COMPRENANT UNE PARTIE DE LIAISON

(30) Priorität: 04.10.2016 DE 102016118765
(43) Veröffentlichungstag der Anmeldung: 14.08.2019
(73) Patentinhaber: Romedis GmbH, 83115 Neubeuern (DE)
(72) Erfinder: RADSPIELER, Andreas, 83115 Neubeuern (DE)
(74) Vertreter: Bobbert & Partner Patentanwälte PartmbB
(86) Internationale Anmeldenummer: PCT/EP2017/074968
(87) Internationale Veröffentlichungsnummer: WO 2018/065362

(56) Entgegenhaltungen:
- WO-A1-2016/135320
- US-A- 5 314 497
- US-A1- 2001 039 159
- US-A1- 2015 118 338

## Beschreibung

Die vorliegende Erfindung betrifft eine Vorrichtung gemäß dem Oberbegriff des Anspruchs 1.

Beinamputierte Personen können mittels Beinprothesen Mobilität zurückgewinnen. Moderne Beinprothesen umfassen verschiedene Module, zu ihnen zählt der Prothesenschaft.

Die vorliegende Erfindung betrifft das technische Gebiet des Herstellens eines Gipsabdrucks als Pause, Vorlage oder Modell für den späteren Prothesenschaft einer Prothese, vorzugsweise für die unteren Extremitäten, also für eine Beinprothese, insbesondere einer Unterschenkelprothese.

In der US 5 314 497 A, aus welcher die Merkmale der Präambel des Anspruchs 1 hervorgehen, sind eine Vorrichtung und ein Verfahren zum Abdichten eines Liners an einer Prothese offenbart.

Eine Aufgabe der vorliegenden Erfindung kann es sein, eine Vorrichtung zur Verwendung beim Fertigen eines Gipsabdrucks für einen Prothesenschaft, oder zumindest für einen Außenschaft hiervon, insbesondere für die untere Extremität, vorzuschlagen.

Die erfindungsgemäße Aufgabe kann durch eine Vorrichtung mit den Merkmalen des Anspruchs 1 gelöst werden.

Erfindungsgemäß wird somit eine medizinische Vorrichtung (kurz: Vorrichtung) vorgeschlagen, welche bei der Anfertigung eines Gipsabdrucks eines Körpergliedstumpfs, insbesondere eines Unterschenkelstumpfs, verwendet werden kann. Diese kann beispielsweise als Grundlage beim Fertigen eines Unterschenkelprothesenschaftes, am nur vorzugsweise stehenden Patienten, verwendet werden.

Dabei weist die Vorrichtung einen Fluidbehälter oder Druckbehälter mit genau oder wenigstens einer Druckkammer oder genau oder wenigstens einer Fluidkammer auf. Diese kann ein optional unter Druck stehendes Fluid aufnehmen oder speichern. Der Druck liegt dabei oberhalb des Atmosphärendrucks. Bei dem Fluid handelt es sich um ein Gas oder eine Flüssigkeit, vorzugsweise Luft bzw. Wasser, da letztere jeweils billig und leicht verfügbar sind.

Der Druckbehälter weist eine Wandung auf, welche aus wenigstens oder genau einem ersten Material gefertigt ist oder wenigstens ein erstes Material aufweist.

Die Wandung des Druckbehälters begrenzt dessen Inneres. Unter dem Inneren des Druckbehälters wird erfindungsgemäß jener Raum oder jenes Volumen verstanden, welcher/s durch die Geometrie des Druckbehälters vorgegeben oder durch eine äußere Wandung des Druckbehälters umfasst oder umschrieben ist. Ist der Druckbehälter beispielsweise zylindrisch, so ist das Innere des Druckbehälters der von der zylindrischen Mantelfläche und den beiden Stirnflächen oder Stirnebenen begrenzte Raum. Ist der Druckbehälter in einem anderen Beispiel rechteckig, so wird der Raum des Inneren durch das Ergebnis der Multiplikation von Höhe, Breite und Tiefe des Rechtecks definiert. Es spielt bei der Bestimmung des Inneren keine Rolle, ob der Raum, der dem Inneren entspricht, fluiddicht begrenzt ist oder nicht. Das Innere beschreibt keinen fluiddicht abgeschlossenen Raum sondern ein von der Wandung umschriebenes Volumen. Jener Raum, welcher nicht zum Inneren des Druckbehälters zählt, wird hierin als dessen Äußeres bezeichnet.

Der Druckbehälter weist in einer ersten Stirnseite eine Einführöffnung auf, durch welche hindurch der Körpergliedstumpf (gemeint sein kann hiermit in diesem Zusammenhang stets auch das distale Stumpfende anstelle des gesamten Stumpfs) in das Innere des Druckbehälters eingeführt werden kann. Die Einführöffnung kann beispielsweise eine offene Stirnfläche oder Stirnebene, eine Durchgangsöffnung in der Wandung oder eine Öffnung sein, welche die Wandung durchbricht oder unterbricht. Im Bereich der Einführöffnung ist das Innere somit nicht durch einen Abschnitt der Wandung von dem Äußeren des Druckbehälters getrennt. Die Einführöffnung kann in einer Eintrittsöffnung oder in einer Einführebene liegen, durch welche der Körpergliedstumpf in das Innere des Druckbehälters eingebracht wird.

Ferner weist der Druckbehälter wenigstens eine oder genau eine fluiddichte Membran auf. Alternativ weist der Druckbehälter keine solche und im Folgenden weiter beschriebene Membran, sondern nur entsprechend geeignete und/oder vorgesehene Aufnahmeeinrichtungen (wie den im Folgenden als optional beschriebenen Verbinder) zum Aufnehmen der Membran am Druckbehälter auf.

Die Membran ist aus einem zweiten Material gefertigt oder weist ein solches auf. Das erste und das zweite Material unterscheiden sich voneinander.

Wenigstens ein Abschnitt der Membran, welcher in einem oberen und/oder einem proximalen Bereich liegt und/oder welcher der Einführöffnung des Druckbehälters zugewandt ist, ist mit wenigstens einem Verbinder verbunden, vorzugsweise lösbar. Mittels dieses wenigstens eines Verbinders ist die Membran, vorzugsweise lösbar, am Druckbehälter befestigt.

Die vorliegende Erfindung betrifft gleichwertig eine medizinische Vorrichtung zur Verwendung bei der Anfertigung eines Gipsabdrucks oder eines Datenmodells eines Körpergliedstumpfs, insbesondere eines Unterschenkelstumpfs, wobei die Vorrichtung einen, Fluidbehälter (kann ein Druckbehälter sein) für eine Flüssigkeit, etwa Wasser, aufweist. Der Fluidbehälter weist erfindungsgemäß eine im Gebrauch der Vorrichtung obere, oder erste, Stirnseite und eine im Gebrauch untere, oder zweite, Stirnseite auf. Im Bereich der zweiten Stirnseite kann der Fluidbehälter fluiddicht verschlossen sein, beispielsweise mittels einer Bodenfläche. Die Vorrichtung weist eine Folie oder Membran zum Aufnehmen eines Abschnitts eines Körpergliedstumpfs auf. Die Membran ist fluiddicht mit dem Behälter verbunden und/oder verschließt diesen wenigstens oben oder an wenigstens der erste Stirnseite, zumindest abschnittsweise, fluiddicht. Der Fluidbehälter kann optional eine Druckkammer aufweisen. Der Fluidbehälter weist optional wenigstens einen Auslass für das Fluid auf, welcher optional mit einem Ventil oder einem Sperrhahn zum Öffnen und Schließen des Auslasses versehen ist. Die Membran ist vorzugsweise sackartig (also mit einem toten Ende wie ein Sack, im Gegensatz zu einem oben und unten offenen Schlauch). Wenigstens ein oberer und/oder proximaler Abschnitt und/oder ein Abschnitt, welcher im Bereich liegt, welcher der Einführöffnung des Druckbehälters zugewandt ist, ist mittels wenigstens eines Verbinders an oder mit einem Abschnitt einer Wandung des Fluidbehälters, beispielsweise einer Bodenfläche oder Seitenfläche hiervon, vorzugsweise lösbar, direkt oder indirekt, befestigt. Die Membran ist vorzugsweise aus einem Material gefertigt oder weist ein solches auf, welches wenigstens in einer ersten Richtung des Materials, vorzugsweise in einer Längseinrichtung des Behälters oder in einer Einführrichtung des Körpergliedstumpfs, keine (oder eine nur geringe) Elastizität oder vorzugsweise keine (oder eine nur geringe) Dehnbarkeit aufweist. Anstatt die vorstehend genannte Membran und/oder den Verbinder aufzuweisen, kann die Vorrichtung ausgestaltet oder konfiguriert sein, um mit einer Membran und/oder einem Verbinder verbunden zu werden. Hierzu geeignete Verbindungseinrichtungen, etwa an einer Bodenfläche oder Seitenwand, können vorgesehen sein.

Erfindungsgemäße Ausführungsformen jeder der vorgenannten Gegenstände können eines oder mehrere der im Folgenden genannten Merkmale in beliebiger Kombination aufweisen, sofern eine konkrete Kombination für den Fachmann nicht als offenkundig technisch unmöglich erkennbar ist. Auch die Gegenstände der Unteransprüche geben jeweils erfindungsgemäße Ausführungsformen an.

Bei allen oben stehenden und/oder folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin räumliche Bezüge wie "oben", "unten", "oberen" oder "unteren" genannt werden, so versteht der Fachmann diese im Zweifel als eine räumliche Angabe mit Bezug auf die Ausrichtung in den hier beigefügten Figuren und/oder die Anordnung der erfindungsgemäßen Vorrichtung(en) in ihrem bestimmungsgemäßen Gebrauch.

Die Membran kann optional mehrere Segmente oder Abschnitte aufweisen. Beispielsweise kann ein distaler Abschnitt, insbesondere ein Abschnitt, auf den niedrige Kräfte, insbesondere geringere Kräfte gegenüber anderen Abschnitten der Membran, ausgeübt werden, geringere Wandstärken gegenüber dem oder den anderen Abschnitten aufweisen. Ein Abschnitt der Membran, auf den niedrigere Kräfte wirken, kann ein anderes, beispielsweise weicheres Material aufweisen gegenüber anderen Abschnitten der Membran. Die Kräfte können Zugkräfte und/oder Druckkräfte sein. Ein weiterer, proximaler Abschnitt der Membran kann aus einem zweiten Material gefertigt sein, oder weist ein solches auf, wobei der proximale Abschnitt gegenüber dem distalen Abschnitt eine höhere Wandstärke und/oder andere Materialeigenschaften aufweisen kann. Insbesondere kann der proximale Abschnitt aus einem Verbundmaterial hergestellt sein oder ein solches aufweisen. Der distale Abschnitt kann einseitig oder beidseitig beschichtet sein.

Mehrere Abschnitte der Membran können materialschlüssig bzw. stoffschlüssig miteinander verbunden sein. Alternativ oder ergänzend können die Abschnitte der Membran mechanisch verbunden sein, beispielsweise mittels Adaptern, Klammern, Klemmringen oder ähnlichem.

In gewissen erfindungsgemäßen, beispielhaften Ausführungsformen ist die Druckkammer ein abgeschlossener und/oder abschließbarer Raum, in welchem das Fluid einem Druck oberhalb des Atmosphärendrucks (kurz: Atmosphäre) ausgesetzt werden kann, ohne aus diesem Raum entweichen zu können.

In manchen erfindungsgemäßen, beispielhaften Ausführungsformen ist die Membran angeordnet, um die Druckkammer zu bilden oder, alternativ, um sie zu begrenzen, beispielsweise indem sie Teil, insbesondere elastischer oder in nur einer Richtung elastischer Teil, der Wand oder Begrenzung der Druckkammer ist.

In gewissen erfindungsgemäßen, beispielhaften Ausführungsformen dient die Membran dazu, um, für sich allein (z. B. in Form eines Ballons) oder alternativ gemeinsam mit Abschnitten der Wandung, eine zumindest teilweise oder vollständig im Inneren des Druckbehälters liegende, fluiddicht abgeschlossene Fluidkammer des Druckbehälters zu bilden. Da sie Fluid unter einem Druck aufnehmen und/oder halten kann, welcher oberhalb Atmosphärendruck liegt, wird diese Fluidkammer hierin als Druckkammer bezeichnet.

Die Begriffe "Fluidkammer" und "Druckkammer" sind in gewissen erfindungsgemäßen, beispielhaften Ausführungsformen und/oder in solchen Ausführungsformen, in denen für den Fachmann aus technischer Sicht nichts dagegen spricht, austauschbar. Was hierin zur "Druckkammer" gesagt ist, kann auch für eine "Fluidkammer" zutreffen.

In manchen erfindungsgemäßen, beispielhaften Ausführungsformen liegt die Druckkammer dann innerhalb des Inneren des Druckbehälters, wenn der in der Druckkammer herrschende Druck einen gewissen Druck nicht überschreitet. Die Druckkammer erstreckt sich hiervon abweichend in gewissen erfindungsgemäßen, beispielhaften Ausführungsformen auch auf das Äußere des Druckbehälters, wenn der in der Druckkammer herrschende Druck die Membran derart beaufschlagt, dass sich letztere beispielsweise durch die Einführöffnung hindurch nach außen, also in das Äußere des Druckbehälters, vorwölbt. Die Druckkammer kann somit ein variables Volumen aufweisen, welches von dem in der Druckkammer herrschenden Druck abhängt. Letzteres trifft nicht auch auf das konstante Innere oder Innenvolumen des Druckbehälters zu.

In manchen erfindungsgemäßen, beispielhaften Ausführungsformen kann in der Druckkammer ungeachtet der die Wandung durchbrechenden Einführöffnung ein Fluid unter Druck gehalten werden, welcher oberhalb der Atmosphäre liegt.

In gewissen erfindungsgemäßen, beispielhaften Ausführungsformen dient die Druckkammer dem Aufnehmen oder dem Abstützen des in das Innere des Druckbehälters eingeführten distalen Endes des Körpergliedstumpfs des Patienten. Die Membran schmiegt sich aufgrund des in der Druckkammer enthaltenen Fluids lateral oder umfangsseitig an das distale Ende des Körpergliedstumpfs oder an den gesamten Körpergliedstumpf an. Auf diese Weise kann es möglich sein, den Körpergliedstumpf im Bereich der gesamten Gipsbinde durch die Membran hindurch mit - bezogen auf eine Flächeneinheit - vorzugsweise unverändertem oder gleichem Druck zu beaufschlagen. Letzteres kann einen vorteilhaften Beitrag der vorliegenden Erfindung darstellen, da bereits die gleichmäßige Druckbeaufschlagung zu einer gleichmäßigen Modellierung der feuchten Gipsbinde führen kann. Das Anliegen der Membran unter dem in der Druckkammer gespeicherten Fluid kann eine Modellierung von Hand vorteilhaft entbehrlich machen oder den Aufwand hierfür deutlich reduzieren. Die Gipsbinde kann eine handelsübliche sein.

In manchen erfindungsgemäßen, beispielhaften Ausführungsformen ist die Druckkammer ein fluiddicht geschlossener Raum, welcher vollständig, oder unter anderem, durch die Wandung des Druckbehälters und die Membran gebildet oder begrenzt wird. In einigen erfindungsgemäßen, beispielhaften Ausführungsformen kann der Begriff "Druckkammer" durch die vorstehende Definition ersetzt werden.

In manchen erfindungsgemäßen, beispielhaften Ausführungsformen ist der Druckbehälter jener Behälter oder Raum, in welchem die Druckammer angeordnet ist.

In einigen erfindungsgemäßen, beispielhaften Ausführungsformen ist der Druckbehälter ein Wasserbehälter.

In manchen erfindungsgemäßen, beispielhaften Ausführungsformen kann der Begriff "Druckbehälter" durch den Begriff "Fluidbehälter" oder "Wasserbehälter" ersetzt werden.

In gewissen erfindungsgemäßen, beispielhaften Ausführungsformen hat der Druckbehälter eine zylindrische Form.

In manchen erfindungsgemäßen, beispielhaften Ausführungsformen ist die Druckkammer derart ausgestaltet und/oder angeordnet, dass der in ihr herrschende Druck, unter anderem oder ausschließlich, von der Einführtiefe des Körpergliedstumpfs in das Innere des Druckbehälters abhängt, jedenfalls im bestimmungsgemäßen Gebrauch der Vorrichtung und bei geschlossenen Ein- und Auslässen, soweit vorhanden.

In gewissen erfindungsgemäßen, beispielhaften Ausführungsformen ist die Membran, beispielsweise ein zweiter Abschnitt der Membran, insbesondere ein Abschnitt mit einer erhöhten Festigkeit (beispielsweise mittels einer erhöhten Wandstärke und/oder anderen Materialeigenschaften durch Verbundmaterialien, Beschichtungen oder ähnlichem) gegenüber dem oder den anderen Abschnitten der Membran, im Bereich der Einführöffnung angeordnet und optional dort direkt oder indirekt mit der Wandung lösbar oder unlösbar verbunden, vorzugsweise fluiddicht. Sie verschließt die Einführöffnung, vorzugsweise ähnlich einem Deckel, soweit der in der Druckkammer liegende Druck nicht wesentlich vom Atmosphärendruck abweicht.

In einigen erfindungsgemäßen, beispielhaften Ausführungsformen ist die Membran angeordnet, um einen Fluid- oder Stoffaustausch im Inneren des Druckbehälters in dessen axialer Richtung zu unterbinden.

In gewissen erfindungsgemäßen, beispielhaften Ausführungsformen ist die Membran in axialer und/oder radialer Richtung stets einlagig angeordnet.

In einigen erfindungsgemäßen, beispielhaften Ausführungsformen ist die Membran an einer ersten Stirnseite des Druckbehälters mit diesem direkt oder indirekt verbunden, nicht aber auch an einer zweiten, der ersten gegenüberliegenden Stirnseite, jedenfalls nicht ohne zwischen Membran und Stirnseite angeordneten Verbinder vorzugsweise von mindestens 5 cm Länge.

In gewissen erfindungsgemäßen, beispielhaften Ausführungsformen ist die Membran flach oder ballonartig (d. h. an einem Ende offen), nicht aber schlauchartig (d. h. an beiden Enden offen).

In einigen erfindungsgemäßen, beispielhaften Ausführungsformen ist die Membran, wenigstens teilweise, vorzugsweise an ihrem Rand, als ein Dichtelement ausgestaltet, etwa als ein Dichtring. Sie wird in diesen Ausführungsformen, beispielsweise im Bereich der Einführöffnung, um den Körpergliedstumpf herum angeordnet, Sie verhindert optional, dass das Fluid, welches in der Druckkammer herrscht, an Druck entlang des Körpergliedstumpfs verliert. Sie mag vorteilhafter Weise einen Austritt von Fluid in das Äußere der Druckkammer und damit einen Druckabfall im Inneren der Druckkammer verhindern.

In manchen erfindungsgemäßen, beispielhaften Ausführungsformen ist die Membran, zumindest beim Gebrauch der Vorrichtung (also bei in das Innere des Druckbehälters eingeführtem Körpergliedstumpf des Patienten) und zumindest in Abschnitten hiervon, im Inneren des Druckbehälters angeordnet. Vorzugsweise liegt sie nur und/oder stets im Inneren des Druckbehälters vor. Alternativ oder ergänzend ist sie direkt oder indirekt in fluiddichter Verbindung mit Abschnitten der Wandung des Druckbehälters verbunden.

In einigen erfindungsgemäßen, beispielhaften Ausführungsformen liegt die Membran auch beim Gebrauch der Vorrichtung (also bei in das Innere des Druckbehälters eingeführtem Körpergliedstumpf des Patienten) ausschließlich im Inneren des Druckbehälters vor.

In manchen erfindungsgemäßen, beispielhaften Ausführungsformen steht die Membran nicht aus dem Inneren des Druckbehälters hervor, insbesondere nicht im Bereich einer zweiten Stirnseite oder im Bereich der Bodenfläche.

In manchen erfindungsgemäßen, beispielhaften Ausführungsformen ist die Membran dauerhaft mit der Vorrichtung verbunden. Dauerhaft bedeutet in gewissen erfindungsgemäßen Ausführungsformen, dass die Membran nicht ohne Einsatz von Werkzeug oder nur zerstörend vom Druckbehälter gelöst werden kann; sie kann beispielsweise mittels Klemmrings oder Klemmringe (nicht zu verwechseln mit Verbindern im Sinne der vorliegenden Erfindung, der zusätzlich vorgesehen sein kann) und einer oder mehrerer Schrauben mit der Wandung dauerhaft und doch lösbar verbunden sein. Eine Lösbarkeit mittels Werkzeug kann vorteilhaft vorgesehen sein, um ein Austauschen der Membran, etwa aufgrund von Verschleiß nach einer Vielzahl von Verwendungen, zu ermöglichen. Sie soll in diesen Ausführungsformen allerdings nicht durch einfaches Überstülpen, Herunterziehen oder dergleichen vom Druckbehälter lösbar sein. Zugleich kann die dauerhafte Befestigung vorteilhaft sicherstellen, dass die bei der Benutzung der Vorrichtung in der Druckkammer über das Fluid auf die Membran übertragenen Kräfte die Membran nicht vom Druckbehälter oder von dessen Wandung lösen können.

In einigen erfindungsgemäßen, beispielhaften Ausführungsformen weist die Vorrichtung keine andere axiale Aufnahme für das freie Stumpfende als die Membran und/oder keine "*axial reference compliant means"* auf. Das Stumpfende kommt vorzugsweise nur mit der Membran in Kontakt. In anderen erfindungsgemäßen Ausführungsformen kann dies anders sein.

In manchen erfindungsgemäßen, beispielhaften Ausführungsformen weist die erfindungsgemäße Vorrichtung keine, insbesondere kreisförmige, und/oder scheibenförmige Abdeckung der Einführöffnung auf, welche beispielsweise aus Gummi gefertigt ist und/oder welche optional einstückig mit einem integralen zentralen Loch ist. In anderen Ausführungsformen ist dies anders; in solchen Ausführungsformen weist die erfindungsgemäße Vorrichtung wenigstens eine, insbesondere kreisförmige, und/oder scheibenförmige Abdeckung der Einführöffnung auf, welche beispielsweise aus Gummi gefertigt ist und/oder welche optional einstückig mit einem integralen zentralen Loch ist.

In einigen erfindungsgemäßen, beispielhaften Ausführungsformen weist die erfindungsgemäße Vorrichtung, insbesondere in ihrem Gebrauch, keinen Sand, kein Gipsmaterial, kein aushärtendes Material auf, insbesondere nicht in der Druckkammer oder zwischen Wandung und Membran.

In gewissen erfindungsgemäßen, beispielhaften Ausführungsformen ist das Fluid nicht Sand, nicht feste Partikel und nicht Kugeln, insbesondere keine Polystyrenkugeln, oder weist derartiges auf.

In gewissen erfindungsgemäßen, beispielhaften Ausführungsformen ist die Membran nicht aus Polyethylen oder weist kein Polyethylen auf.

In manchen erfindungsgemäßen, beispielhaften Ausführungsformen weist die erfindungsgemäße Vorrichtung keine Einrichtung wie einen elastischen Ring oder einen Gummiring und insbesondere keinen Gummiring auf, welcher zur Befestigung der Membran an einer äußeren Wandung des Druckbehälters vorgesehen ist. In anderen erfindungsgemäßen Ausführungsformen kann dies anders sein. Ein solcher Gummiring wäre keine Verbindung im Sinne der vorliegenden Erfindung.

In einigen erfindungsgemäßen, beispielhaften Ausführungsformen weist die erfindungsgemäße Vorrichtung keine Vakuumquelle auf (insbesondere keine Vakuumquelle, welche elektrisch oder hydraulisch betrieben wird) oder ist mit einer solchen nicht in Fluidverbindung verbunden.

In bestimmten erfindungsgemäßen, beispielhaften Ausführungsformen weist die erfindungsgemäße Vorrichtung keine Druckquelle und/oder keine, insbesondere aufblasbaren, Aufweitevorrichtungen oder anderweitige *"expander means"* auf oder ist mit einer solchen nicht verbunden.

Die erfindungsgemäße Vorrichtung weist erfindungsgemäß keinen Unterdruckanschluss auf.

In gewissen erfindungsgemäßen, beispielhaften Ausführungsformen weist die erfindungsgemäße Vorrichtung keine Luftkammer auf.

In manchen erfindungsgemäßen, beispielhaften Ausführungsformen weist die erfindungsgemäße Vorrichtung keine Förderschrauben auf.

In einigen erfindungsgemäßen, beispielhaften Ausführungsformen ist die Vorrichtung ausgestaltet, um mit ihr einen negativen Abdruck des Körpergliedstumpfs anzufertigen. Der negative Abdruck hat eine Wandstärke von vorzugsweise 2 bis 8mm, besonders bevorzugt von 3 bis 6 mm.

In manchen erfindungsgemäßen, beispielhaften Ausführungsformen ist die Vorrichtung nicht ausgestaltet, um mit ihr einen positiven Abdruck des Körpergliedstumpfs anzufertigen.

Erfindungsgemäß ist der wenigstens eine Verbinder an einer Bodenfläche, an einer Seitenfläche der Wandung und/oder an der zweiten Stirnseite des Druckbehälters befestigt, vorzugsweise mit einem seiner Enden oder Endabschnitte, während das andere Ende oder der andere Endabschnitt vorzugsweise mit der Membran verbunden ist.

Der Verbinder kann mittels dieser Befestigung die Membran im Inneren des Druckbehälters fixieren, sodass die Membran, insbesondere zum vorteilhaft einfachen Einführen eines Körpergliedstumpfes in das Innere des Druckbehälters, sich nicht durch die Einführöffnung nach außen wölben oder stülpen kann.

Der Verbinder kann mittels mechanischen Befestigungsmittels, beispielsweise mittels Haken, Klemmverbindung, Schrauben, Nieten oder ähnlichem an der Bodenfläche, an der Wandung und/oder an der zweiten Stirnseite des Druckbehälters befestigt sein.

In einigen erfindungsgemäßen, beispielhaften Ausführungsformen ist der wenigstens eine Verbinder wenigstens 10 cm, in anderen wenigstens 20 cm, in weiteren wenigstens 30 cm lang.

In einigen erfindungsgemäßen, beispielhaften Ausführungsformen dient der wenigstens eine Verbinder nicht zum Verbinden der Membran in einem Bereich des oberen Randes der Wandung des Druckbehälters und/oder nicht zum Herstellen einer fluiddichten Verbindung zwischen Membran und Druckbehälter.

In manchen erfindungsgemäßen, beispielhaften Ausführungsformen ist der wenigstens eine Verbinder mit einem verstärkten Abschnitt der Membran verbunden. Ein verstärkter Abschnitt der Membran kann beispielsweise eine erhöhte Wandstärkte, andere Materialien und/oder Materialbeschichtungen aufweisen.

Ein verstärkter Abschnitt der Membran kann beispielsweise eine höhere Festigkeit aufweisen. Mittels des verstärkten Abschnitts der Membran können erhöhte Kräfte, insbesondere Reibungskräfte, auf den verstärkten Abschnitt, beispielsweise bei einem Einführen eines Körpergliedstumpfes in, oder einem Herausziehen aus dem Inneren des Druckbehälters der Vorrichtung vorteilhaft Kräfte über den Verbinder abgeleitet werden.

Ein weiterer Abschnitt der Membran, der nicht, zumindest nicht vollständig, diesen Kräften ausgesetzt ist, kann andere Materialeigenschaften aufweisen, beispielsweise um einen angenehmeren und weicheren Sitz eines Körpergliedstumpfes in der Membran in der Druckkammer während der Anfertigung eines Gipsabdrucks zu ermöglichen. Insbesondere die distalen Bereiche eines Körpergliedstumpfes sind oft besonders druckempfindlich und sollten daher während der Anfertigung eines Gipsabdrucks möglichst druckentlastet werden.

In manchen erfindungsgemäßen, beispielhaften Ausführungsformen ist der verstärkte Abschnitt der Membran einerseits mit dem wenigstens einen Verbinder und andererseits mit der Wandung oder dem Boden des Druckbehälters verbunden.

In manchen erfindungsgemäßen, beispielhaften Ausführungsformen sind wenigstens zwei Verbinder am Umfang der Membran befestigt. Ebenso können drei, vier, fünf, sechs oder mehr Verbinder am Umfang der der Membran befestigt sein. Die Verbinder können in einem regelmäßigen oder in einem unregelmäßigen Abstand zueinander am Umfang der der Membran befestigt sein.

In gewissen erfindungsgemäßen, beispielhaften Ausführungsformen weist der Druckbehälter die Einführöffnung sowie optional einen oder mehrere Ein- und/oder Auslässe auf, welche jedoch allesamt mit einer oder jeweils einer Verschlusseinrichtung zu deren fluiddichtem Verschließen versehen sind. Ansonsten ist die Wandung des Druckbehälters oder der Druckkammer in diesen Ausführungsformen fluiddicht.

In einigen erfindungsgemäßen, beispielhaften Ausführungsformen sind die mit einer Verschließeinrichtung versehenen Ein- und/oder Auslässe Teil der Wandung.

In gewissen erfindungsgemäßen, beispielhaften Ausführungsformen ist die Druckkammer ausschließlich durch die Membran und Teile oder Abschnitte der Wandung, oder ausschließlich durch Membran und Teile oder Abschnitte der Wandung und fluiddichte Verbindungen zwischen Membran und Wandung ausgestaltet.

In einigen erfindungsgemäßen, beispielhaften Ausführungsformen zählt eine Stirnseite des Druckbehälters zur Wandung, insbesondere die hierin als zweite Stirnseite bezeichnete Stirnseite.

In manchen erfindungsgemäßen, beispielhaften Ausführungsformen ist der Druckbehälter an seiner zweiten Stirnseite fluiddicht verschlossen oder verschließbar.

Der Druckbehälter kann eine erste und eine zweite Stirnseite aufweisen. Die Einführöffnung kann eine stirnseitige Öffnung sein. Sie liegt vorzugsweise in der ersten Stirnseite oder im Bereich der ersten Stirnseite.

Die Membran kann lösbar oder nicht lösbar mit der Wandung oder einem anderen Abschnitt des Druckbehälters verbunden sein.

In einigen erfindungsgemäßen, beispielhaften Ausführungsformen weist die medizinische Vorrichtung wenigstens einen Auslass auf, welcher eine Fluidverbindung zwischen der Druckkammer und dem Äußeren des Druckbehälters ist oder ermöglicht. Ferner weist sie ein Ventil, einen Sperrhahn oder eine andere Verschlussvorrichtung zum reversiblen Schließen des Auslasses oder der Fluidverbindung auf.

Der Auslass kann vorteilhaft genutzt werden, um den in der Druckkammer vorliegenden Druck durch Ablassen von Fluid aus der Druckkammer zu senken. Dies kann zum Einführen des Körpergliedstumpfs erforderlich oder hilfreich sein, oder zum Einstellen einer Einführtiefe des Stumpfs.

In einigen erfindungsgemäßen, beispielhaften Ausführungsformen weist der Druckbehälter eine erste und eine zweite Stirnseite auf. Dabei ist der Auslass des Druckbehälters optional im Bereich der zweiten Stirnseite oder eines Stirnendes des Druckbehälters angeordnet.

In einigen erfindungsgemäßen, beispielhaften Ausführungsformen ist der Druckbehälter mit dem Auslass oder mit einem hiervon getrennt vorliegenden Einlass mit der Druckquelle verbunden. Es können somit zwei Durchlässe in der Wandung vorliegen, nämlich Auslass und Einlass.

In einigen erfindungsgemäßen, beispielhaften Ausführungsformen weist die Druckkammer des Druckbehälters in seinem/ihrem Inneren Wasser oder eine andere Flüssigkeit auf oder sind hiermit gefüllt. Eine Flüssigkeit führt aufgrund ihrer fehlenden Komprimierbarkeit zu reproduzierbareren Ergebnissen beim Fertigen des Gipsabdrucks. Wasser ist dabei die billigste und am einfachsten verfügbare Variante.

In gewissen erfindungsgemäßen, beispielhaften Ausführungsformen ist die Wandung des Druckbehälters zumindest in Abschnitten hiervon transparent. Dies kann eine visuelle Überwachung der Einführtiefe und anderer Aspekte beim Fertigen des Gipsabdrucks durch den Orthopädietechniker auf einfache Weise erlauben.

In manchen erfindungsgemäßen, beispielhaften Ausführungsformen weisen der Druckbehälter im Bereich wenigstens eines transparenten Abschnitts hiervon eine oder mehrere Markierungen auf. An ihnen kann die Einführtiefe des Körpergliedstumpfs abgelesen werden.

In einigen erfindungsgemäßen, beispielhaften Ausführungsformen weist der Druckbehälter einen mehrteiligen Dichtring, insbesondere einen Oberschenkeldichtring (nicht zu verwechseln mit einem Verbinder im Sinne der vorliegenden Erfindung) auf, in anderen Ausführungsformen weist er keinen solchen Ring auf. Dieser ist, falls vorgesehen, am Druckbehälter im Bereich der Einführöffnung oder eines Klemmrings bzw. eines Befestigungsrings, welcher die Membran trägt, lösbar verbindbar, beispielsweise anschraubbar. Er kann ein Auftreiben der Membran in ein Äußeres des Druckbehälters aufgrund des jenseits der Membran herrschenden Drucks verhindern oder begrenzen. Dies kann zu korrekteren Druckverhältnissen und Ergebnissen des Abdrucks führen. Zudem kann die Membran vor einem Abscheren beispielsweise am Rand der Einführöffnung oder einem anderweitigen Verletzen der Membran, etwa im Spalt zwischen Körpergliedstumpf und Rand der Einführöffnung, bewahrt werden. Ein oder mehrere Verbinder im Sinne der vorliegenden Erfindung können zusätzlich vorgesehen sein.

In manchen erfindungsgemäßen, beispielhaften Ausführungsformen ist die Druckkammer durch wenigstens einen Abschnitt der Wandung und durch die Membran gebildet oder begrenzt. Die Membran ist mit dem Abschnitt der Wandung, vorzugsweise im Bereich der zweiten Stirnseite des Druckbehälters, stoffschlüssig, kraftschlüssig und/oder formschlüssig verbunden. Hierzu kann ein Verbinder, oder eine Verbindungseinrichtung, vorgesehen sein. Mittels des Verbinders kann in einigen erfindungsgemäßen Ausführungsformen ein ungewünschtes Auswölben, Wandern, Auftreiben oder Erstrecken der Membran noch oben oder in ein Äußeres des Druckbehälters, in welchem die Membran - anders als im Inneren des Druckbehälters - nicht durch die Wandung seitlich gestützt wird, verhindert oder auf ein als zulässig erachtetes Maß beschränkt werden. Der Verbinder hält die Membran somit, zumindest im Wesentlichen, optional im Inneren des Druckbehälters. Dies, oder der Verbinder, kann einem unerwünschten Auftreiben des Körpergliedstumpfs entgegenwirken. Das Auftreiben kann zu einer ungünstigen Beeinflussung des in der Druckkammer und mittels der Membran auf den Körpergliedstumpf herrschenden Druck haben, indem die Membran nicht mehr in allen Abschnitten, in welchen sie den Stumpf umgibt, diesen mit einheitlichem Druck anliegt. Damit aber liegen beim Fertigen des Gipsabdrucks nicht die optimalen Drücke am Stumpf an mit der Folge, dass der gefertigte Gipsabdruck nicht unter späteren Belastungen, die beim Gehen mit der zu fertigenden Prothese auftreten, gefertigt wurde. Ferner kann ein vermindertes oder verhindertes Auftreiben einen Beitrag zum Schutz der Membran leisten, welche im Inneren des Druckbehälters durch dessen Wandung vor Beschädigung geschützt ist. Es bedarf beim Vorsehen des Verbinders erfindungsgemäß vorteilhaft keiner anderweitigen Begrenzung eines Auftreibens der Membran, etwa durch einen eng am Oberschenkel anliegenden Ring. Da ein solcher Ring in einer Vielzahl von Größen vorgehalten werden müsste, um mit der erfindungsgemäßen Vorrichtung Gipsmodelle für eine Vielzahl unterschiedlich dicker Körpergliedstümpfe fertigen zu können, liegt eine für den Fachmann leicht nachzuvollziehende Vereinfachung vor. Sie bedeutet neben einer Vereinfachung des Gebrauchs der Vorrichtung auch eine Einsparung an Material für Ringe, Kosten und dergleichen.

In manchen erfindungsgemäßen, beispielhaften Ausführungsformen ist der Verbinder mit der Membran in einem Abschnitt der Membran verbunden, welcher im Bereich der Einführöffnung liegt.

In bestimmten erfindungsgemäßen, beispielhaften Ausführungsformen ist die Membran im Bereich der Einführöffnung oder der ersten Stirnseite des Druckbehälters mit diesem verbunden, und zusätzlich in einem zweiten, hiervon verschiedenen Bereich oder Abschnitt des Druckbehälters. Der zweite Bereich kann vorzugsweise im Inneren des Druckbehälters und/oder in der Druckkammer liegen. Die Membran kann vorzugsweise mittels des Verbinders mit dem zweiten Bereich verbunden sein. Die Membran kann vorzugsweise ausschließlich indirekt mit dem zweiten Bereich in Kontakt stehen, nämlich optional mittels des Verbinders, also vorzugsweise selber keinen direkt Kontakt mit dem zweiten Bereich haben, diesen vorzugsweise also nicht berühren.

In einigen erfindungsgemäßen, beispielhaften Ausführungsformen ist der zweite Bereich ein Randbereich der Bodenfläche, der zweiten Stirnfläche oder -seite.

In manchen erfindungsgemäßen, beispielhaften Ausführungsformen ist der Verbinder eine elastische Feder oder weist ein elastisches Element auf.

In einigen erfindungsgemäßen, beispielhaften Ausführungsformen ist der Verbinder nicht elastisch oder nicht dehnbar.

In einigen erfindungsgemäßen, beispielhaften Ausführungsformen ist der Verbinder längenverstellbar. Mittels einer entsprechenden Verstelleinrichtung, welche vorzugsweise vom Äußeren des Druckbehälters aus verstellbar ist, kann seine Länge verstellt werden. Hierdurch wird der Abstand zwischen distalem Ende der Membran und beispielsweise der Bodenfläche, unteren Stirnseite oder Stirnfläche des Druckbehälters verändert. Dies erlaubt es, die Membran innerhalb des Druckbehälters veränderlich auszurichten, was ein optimales Einstellen der erfindungsgemäßen Vorrichtung auf den konkreten Körpergliedstumpf ungeachtet seiner Länge erlauben kann.

In manchen erfindungsgemäßen, beispielhaften Ausführungsformen verbindet der Verbinder die Membran, direkt oder indirekt, mit einer Stirnseite des Druckbehälters, welche dem Ende des Druckbehälters, welches die Einführöffnung aufweist, gegenüberliegt, also insbesondere mit einer unteren Stirnseite, Stirnfläche oder Bodenfläche.

In manchen erfindungsgemäßen, beispielhaften Ausführungsformen verbindet der Verbinder die Membran nicht mit einem mittigen oder zentralen Bereich der Stirnfläche, Stirnseite oder Bodenfläche. Dies erlaubt oder begünstigt eine vergleichsweise freie Anordnung des in die Membran eingeführten Körpergliedstumpfs innerhalb des Druckbehälters, ohne dass der Verbinder über die Membran Druck auf distale Abschnitte des Körpergliedstrumpfs ausübt.

In manchen erfindungsgemäßen, beispielhaften Ausführungsformen wird unter einem Verbinden ein form- und/oder kraftschlüssiges und/oder stoffschlüssiges Verbinden verstanden.

In manchen erfindungsgemäßen, beispielhaften Ausführungsformen ist der Verbinder angeordnet, um einen im Gebrauch oberen Abschnitt der Membran zu verbinden.

In manchen erfindungsgemäßen, beispielhaften Ausführungsformen ist der Verbinder angeordnet, um einen Abstand zwischen dem mit dem Verbinder verbundenen Abschnitt der Membran einerseits und dem mit dem Verbinder ebenfalls verbundenen Abschnitts der Bodenfläche, unteren Stirnseite oder Stirnfläche andererseits innerhalb vorgegebener Grenzen zu halten. Der Abstand kann bei nicht elastischem Verbinder beispielsweise konstant sein.

In manchen erfindungsgemäßen, beispielhaften Ausführungsformen ist der Verbinder direkt mit der Wandung verbunden. Der Verbinder kann hierbei das Ergebnis eines Fügeverfahrens sein, etwa ein Klebeabschnitt, ein Niet oder dergleichen.

In manchen erfindungsgemäßen, beispielhaften Ausführungsformen berührt die Membran im Bereich ihrer Verbindung die Bodenfläche, Bodenseite oder unteren Stirnseite, in anderen Ausführungsformen berührt die Membran oder Material der Membran sie nicht.

In gewissen erfindungsgemäßen, beispielhaften Ausführungsformen ist die Membran aus einem Material gefertigt oder weist ein solches auf, welches in einer ersten Richtung des Materials eine andere Elastizität oder Dehnbarkeit des Materials aufweist als in einer von der ersten Richtung verschiedenen zweiten Richtung, wobei die zweite Richtung zur ersten Richtung optional senkrecht stehen kann. In diesen oder anderen erfindungsgemäßen, beispielhaften Ausführungsformen wird die in der ersten und/oder zweiten der beiden Richtungen jeweils spezifische Elastizität oder Dehnbarkeit durch Fasern erreicht, welche die Membran aufweist, welche beispielsweise in Silikon, eine Silikonmatrix oder ein anderes Material, vorzugsweise ein fluiddichtes Material, eingebettet sind. Solche Fasern können in der ersten und/oder zweiten Richtung der Membran verlaufen, im Wesentlichen in der ersten und/oder zweiten Richtung verlaufen und/oder im Wesentlichen in der ersten und/oder zweiten Richtung wirken. In diesen oder anderen erfindungsgemäßen Ausführungsformen werden die unterschiedlichen Elastizitäten oder Dehnbarkeiten ergänzend oder alternativ durch andere Ausgestaltungen erreicht.

Die erste Richtung kann, wenn die Membran gebrauchsfertig oder bestimmungsgemäß am Druckbehälter befestigt ist, eine Einführrichtung des Körpergliedstumpfs oder eine Längsrichtung des Druckbehälters sein. Die zweite Richtung kann im Winkel, z. B. im rechten Winkel, zur ersten Richtung stehen.

Fasern, welche sich in der ersten Richtung erstrecken oder verlaufen oder wirken, sind optional nicht oder in nur geringem Umfang dehnbar. Optional sind sie weniger dehnbar als optional vorgesehene Fasern, welche in der zweiten Richtung verlaufen, sich erstrecken oder wirken. Fasern, welche in der zweiten Richtung verlaufen, wirken oder sich erstrecken, können, sofern vorgesehen, dehnbar sein, sie können dehnbarer oder elastischer sein als jene Fasern der ersten Richtung; ihre Dehnbarkeit kann optional jener der Fasern der in erster Richtung verlaufenden Fasern entsprechen. Die in der ersten Richtung verlaufenden Fasern sind optional, ebenso die in der zweiten Richtung verlaufenden Fasern. Die Dehnbarkeit oder Elastizität der Fasern kann der Dehnbarkeit oder Elastizität der Membran entsprechen oder hierzu korrelieren. Die Fasern können aus Nylon sein oder Nylon aufweisen. Sie können aus zugfestem Material sein, insbesondere aus Material, welches zugfester ist als das sie einbettende Material. Eine solche Ausgestaltung, welche beispielsweise eine Längsdehnung (welche optional die erste Richtung sein kann) verhindert oder merklich beschränkt, zugleich aber eine Dehnung der Membran in Umfangs- oder Radialrichtung zu ihrem Anpassen an den Körpergliedstumpf erlaubt, kann ebenfalls einem unerwünschten Auftreiben des Körpergliedstumpfs vorteilhaft entgegenwirken. Zudem kann das Verhältnis zwischen der Tiefe der Druckkammer und der Einführtiefe innerhalb der Membran vorteilhaft gleich bleiben. Zudem kann optional auf den o. g. mehrteiligen Dichtring oder Oberschenkeldichtring verzichtet werden. Ferner kann es möglich sein, mit nur einer Membran erfindungsgemäß Gipsabdrücke an einer ganzen Reihe von im Querschnitt unterschiedlich dicken Körpergliedstümpfen zu fertigen. Die Membran legt sich dabei vorteilhaft sowohl bei dicken als auch bei dünnen Stümpfen ausreichend eng an, um das hierin beschrieben Faltenwerfen der Membran zu vermeiden. Die Quer- oder Umfangsdehnbarkeit, welche die Membran in einigen erfindungsgemäßen Ausführungsformen selbst dann aufweisen kann, wenn die Längsdehnbarkeit verhindert oder beschränkt ist, trägt hierzu vorteilhaft bei.

In einigen erfindungsgemäßen, beispielhaften Ausführungsformen ist die Membran teilweise, in wenigstens einem Abschnitt hiervon oder vollständig bzw. insgesamt weniger als 2 mm, vorzugsweise weniger als 1 mm dick. Bei dieser Dicke verfälscht sie den Unterschied zwischen der tatsächlichen Geometrie des Körpergliedstumpfs und dem Gips in einem allenfalls vernachlässigbaren Maß.

In gewissen erfindungsgemäßen, beispielhaften Ausführungsformen weist der Druckbehälter eine verschließbare Luftablassöffnung mit entsprechender Verschließeinrichtung auf. Aus dieser kann Luft in deren nicht verschlossenem Zustand aus der Druckkammer entweichen. Dies ist beim erstmaligen Befüllen der Druckkammer mit einer Flüssigkeit von Vorteil; die Vorrichtung, sofern sie mit Flüssigkeit befüllt verwendet werden soll, muss nicht mit dieser befüllt erworben und ausgeliefert werden. Sie kann luftgefüllt und damit ungleich leichter transportiert werden. Die vorhandene Luft kann beim Befüllen mit Flüssigkeit aus der verschließbaren Luftablassöffnung ausgelassen werden.

In manchen erfindungsgemäßen, beispielhaften Ausführungsformen weist die medizinische Vorrichtung eine Einrichtung auf, mittels welcher wenigstens der Druckbehälter bezogen auf einen Untergrund, auf welchem die Vorrichtung ruht oder der Patient mit seinem gesunden Bein steht, höhenverstellbar ist.

In gewissen erfindungsgemäßen, beispielhaften Ausführungsformen ist die Wandung des Druckbehälters aus Kunststoff, beispielsweise Plexiglas oder Polycarbonat oder weist eines oder mehrere dieser Materialien auf.

In gewissen erfindungsgemäßen, beispielhaften Ausführungsformen weist die Vorrichtung keinen Rand oder Ring mit einer Öffnung für den Oberschenkel des Patienten auf, welcher ausgestaltet ist, um mit dem Druckbehälter und/oder der Wandung lösbar verbunden zu werden.

In gewissen erfindungsgemäßen, beispielhaften Ausführungsformen weist der Druckbehälter und/oder die Wandung keine Luftöffnung auf.

In bestimmten erfindungsgemäßen, beispielhaften Ausführungsformen entspricht der proximale Abschnitt der Membran dem im Gebrauch der Vorrichtung oberen Abschnitt der Membran.

Der proximale Abschnitt kann die gesamte obere Hälfte umfassen.

Er kann die oberen 0 bis 30 oder 0 bis 40 % der Membran umfassen.

Ein proximaler oder oberer Abschnitt der Membran kann sich vom freien oder mit dem Druckbehälter verbundenen Rand der Membran, an welchem vorbei der Patient mit dem Körpergliedstumpf beim Gebrauch der erfindungsgemäßen Vorrichtung in die Membran einsteigt, über die ersten 10, 20, 30 bis hin zu 40 oder 50% der Gesamtlänge der Membran erstrecken.

Die Gesamtlänge kann wahlweise ab der Höhe, in welcher die Membran mit dem Druckbehälter verklemmt oder verschraubt oder anderweitig verbunden ist, bestimmt sein.

Die Gesamtlänge kann wahlweise ab der Einführöffnung des Druckbehälters bis zum distalen Ende der Membran bestimmt sein.

In bestimmten erfindungsgemäßen, beispielhaften Ausführungsformen weist der Verbinder, die Membran und/oder die Bodenfläche wenigstens ein Gewinde zum Verschrauben der Membran oder eines hiermit verbundenen Elements, direkt oder indirekt, mit dem Druckbehälter auf.

In bestimmten erfindungsgemäßen, beispielhaften Ausführungsformen begrenzt der Verbinder ein durch Fluid in der Druckkammer erzeugtes Auftreiben der Membran derart, dass eine Kontaktpunktfläche zwischen Körpergliedstumpf und Membran auf selber Höhe liegt wie ein Übergangsabschnitt, in welchem die Druckkammer gerade nicht mehr von der Wandung (oder einem hiermit verbundenen Element wie einem Klemmring) sondern von der Membran gebildet wird und/oder die Membran nicht mehr von der Wandung (oder einem hiermit verbundenen Element wie einem Klemmring) - insbesondere, radial, lateral oder seitlich - gestützt wird.

In bestimmten erfindungsgemäßen, beispielhaften Ausführungsformen ist die Membran aus einem Material gefertigt oder weist ein solches auf, welches in einer ersten Richtung und/oder in einer zweiten Richtung des Materials Fasern aufweist, welche in eine Matrix eingebettet oder mit dieser auf andere Weise verbunden sind.

In bestimmten erfindungsgemäßen, beispielhaften Ausführungsformen sind die Membran, welche optional eine Matrix aufweist, oder ihre Matrix, aus Silikon oder weisen Silikon auf.

In bestimmten erfindungsgemäßen, beispielhaften Ausführungsformen haben manche oder alle der Fasern einen gewellten, kurvigen oder zig-zag-Verlauf.

In bestimmten erfindungsgemäßen, beispielhaften Ausführungsformen ist die Membran in einer ersten Richtung hiervon und/oder in einer zweiten Richtung hiervon nicht dehnbar oder nicht elastisch.

In bestimmten erfindungsgemäßen, beispielhaften Ausführungsformen bedeutet "nicht-dehnbar" oder "nichtelastisch", dass der E-Modul des betreffenden Bauteils (Verbinder, Membran, Fasern, usw.) wenigstens oberhalb 700 N/mm², vorzugsweise oberhalb 1000 N/mm², besonders bevorzugt oberhalb 2000 N/mm², liegt.

In bestimmten erfindungsgemäßen, beispielhaften Ausführungsformen bedeutet "nicht-dehnbar" oder "nichtelastisch", dass eine Dehnung des betreffenden Bauteils (Verbinder, Membran, Fasern, usw.) nicht mehr als 20%, bevorzugt nicht mehr als 10%, vorzugsweise nicht mehr als 5%, besonders bevorzugt nicht mehr als 2% seiner Länge betragen darf, bevor das Bauteil reißt oder bricht.

In bestimmten erfindungsgemäßen, beispielhaften Ausführungsformen hat die Membran, Fasern hiervon und/oder der Verbinder einen E-Modul wie Nylon.

In bestimmten erfindungsgemäßen, beispielhaften Ausführungsformen ist ergänzend zum oberen und/oder proximalen Abschnitt der Membran und/oder zu dem Abschnitt der Membran, welcher im Bereich liegt, welcher der Einführöffnung des Druckbehälters zugewandt ist, auch ein unterer und/oder distaler Abschnitt der Membran und/oder ein Abschnitt, welcher im Bereich der Membran liegt, welcher der Einführöffnung des Druckbehälters abgewandt und/oder einer Bodenfläche zugewandt ist, mittels wenigstens eines hierin als einen distalen Verbinder bezeichneten Verbinders an oder mit einem Abschnitt einer Wandung des Fluidbehälters, beispielsweise einer Bodenfläche oder Seitenfläche hiervon, vorzugsweise lösbar, direkt oder indirekt, befestigt.

Das auf Verbinder hierin Ausgeführte trifft, insbesondere was deren Material, Länge, Elastizität, Dehnbarkeit und andere Eigenschaften, insbesondere Materialeigenschaft, betrifft, auch auf den hier als einen distalen Verbinder bezeichneten Verbinder zu, sofern hierdurch kein für den Fachmann erkennbarer Widerspruch entsteht.

Der distale Verbinder kann in beliebigen erfindungsgemäßen Ausführungsformen ausgestaltet und/oder vorgesehen sein, wie in der WO 2016/135320 A1, deren diesbezügliche Offenbarung hiermit ebenfalls mittels Bezugs vollumfänglich zum Gegenstand auch der vorliegenden Anmeldung gemacht wird.

In manchen erfindungsgemäßen, beispielhaften Ausführungsformen liegen der Verbinder und/oder distale Verbindung vollständig und ohne Kontakt zum Äußeren des Druckbehälters in dessen Inneren.

In manchen erfindungsgemäßen, beispielhaften Ausführungsformen weisen der Druckbehälter und/oder eine hiermit kommunizierende Leitung ein Manometer auf, welches den in der Druckkammer herrschenden Druck bestimmt oder misst. Der Orthopädietechniker kann sich beispielsweise für die vorstehend genannte Druckerhöhung am gemessenen Druck orientieren.

In einigen erfindungsgemäßen, beispielhaften Ausführungsformen ist die Membran ein- und/oder ausstülpbar, vorzugsweise elastisch dehnbar. In anderen ist sie in einer Richtung elastisch, in einer hierzu insbesondere senkrechten Richtung hingegen nicht elastisch oder vergleichsweise oder maßgeblich weniger elastisch.

In manchen erfindungsgemäßen, beispielhaften Ausführungsformen ist die Membran angeordnet, um bei Befüllen der Druckkammer mit einem Fluid durch dieses Fluid durch die Einführöffnung hindurch in ein Äußeres des Druckbehälters stülpbar zu sein.

In gewissen erfindungsgemäßen, beispielhaften Ausführungsformen ist die Membran dehnbar, bleibt aber ähnlich einem Finger eines Gummihandschuhs oder einem Luftballon im gedehnten Zustand bis auf eine Öffnung geschlossen.

In einigen erfindungsgemäßen, beispielhaften Ausführungsformen ist die Membran am Druckbehälter angeordnet, um diesen stirnseitig abzudichten. Dies erfolgt vorzugsweise nicht mit dem Verbinder.

In manchen erfindungsgemäßen, beispielhaften Ausführungsformen weist die Vorrichtung in einem Ringraum oder zylindrischen Raum zwischen der, vorzugsweise elastisch dehnbaren, Membran und der Wandung des Druckbehälters keine Mündung eines Anschlussschlauchs auf.

In einigen erfindungsgemäßen, beispielhaften Ausführungsformen weist die Vorrichtung einen Befestigungsring oder Stumpfdichtring auf, welcher beispielsweise wenigstens zwei Halb- oder Teilschalen (kurz: Schalen) aufweist oder hieraus besteht. Der Befestigungsring ist dem Druckbehälter stirnseitig auflegbar (und beispielsweise mittels geeigneter Verbindungselemente, wie rein exemplarisch Schrauben, mit diesem verbindbar), unter Begrenzung der Einführöffnung für den Stumpf. Der Befestigungsring kann optional oder vorzugsweise ein Auftreiben der Membran beim Gebrauch der Vorrichtung verhindern. Ein solcher Befestigungsring oder Stumpfdichtring ist kein Verbinder im Sinne der vorliegenden Erfindung. Ein solcher Ring kann zusätzlich zu einem solchen Verbinder vorgesehen sein.

In einigen erfindungsgemäßen, beispielhaften Ausführungsformen weist die Vorrichtung keinen Schlitten auf, auf welchem der Druckbehälter montiert wäre.

In manchen erfindungsgemäßen, beispielhaften Ausführungsformen weist die Vorrichtung keine elektrisch, hydraulisch und/oder hydraulisch betriebene oder betreibbare Einrichtung zum Erzeugen von Druck, insbesondere angeordnet zum Erzeugen von Druck auf die Membran, auf oder ist ein einer solchen nicht verbunden.

Die erfindungsgemäße Vorrichtung kann mit einem oder mehreren Merkmalen in beliebiger Kombination ausgestattet sein wie sie in der WO 2016/135320 A1 offenbart sind, deren Offenbarung hiermit mittels Bezugs vollumfänglich zum Gegenstand auch der vorliegenden Anmeldung gemacht wird.

In einigen erfindungsgemäßen, beispielhaften Ausführungsformen ist die Membran nicht schlauchförmig, d. h. nicht an beiden Enden hiervon offen.

In gewissen erfindungsgemäßen, beispielhaften Ausführungsformen ist die Membran aus fluiddichtem, insbesondere wasserdichtem Material. Sie kann aus Silikon sein oder Silikon aufweisen. Sie kann aus faserverstärktem Silikon sein oder solches aufweisen. Statt Silikon kann ein Co-Polymer oder ein Gummi vorgesehen sein.

Einige oder alle erfindungsgemäßen Ausführungsformen können einen oder mehrere der oben oder im Folgenden genannten Vorteile aufweisen.

Es liegt auf der Hand, dass der Prothesenschaft dem Patienten aufgrund der zurück gewonnenen Mobilität insbesondere im Stehen und im Gehen von Nutzen ist. Im Stehen und im Gehen, wenn der Schaft bestimmungsgemäß belastet wird, muss der Schaft daher im besonderen Maße bequem sitzen. Die bislang bekannt gewordenen Verfahren zum Fertigen des Gipsabdrucks oder zum Vermessen des Stumpfs werden dem allerdings nicht auf optimale Weise gerecht, da sie die Weichteilverschiebungen im Stumpf etwa relativ zum knöchernen Anteil des Stumpfs, wie sie bei Belastung des Schafts später auftreten, mangels Belastung des Stumpfs während des Vermessens oder des Gipsens nicht berücksichtigen können. Das Ergebnis kann zu einer Ungenauigkeit beim Fertigen des Schaftes führen, welche selbst bei Abnehmen von Körpermaßen in genau definierten Höhen und Bereichen im cm-Bereich liegt. Dies ist bei Verwendung der erfindungsgemäßen Vorrichtung bei stehendem Patienten vorteilhaft nicht der Fall - der Stumpf unterliegt während des Vermessens oder des Gipsens nahezu identischen Belastungen und Weichteilverlagerungen wie in der späteren Belastung im Schaft.

Mittels der Erfindung ist es bei während des Anfertigens des Gipsabdrucks oder des Vermessens stehendem und den Stumpf belastendem Patienten vorteilhaft erstmals möglich, zuverlässig und vor allem auch reproduzierbar einen Gipsabdruck des Stumpfs des Patienten zu fertigen oder den Stumpf zu vermessen um ein Datenmodell, welches den Stumpf widerspiegelt, zu erhalten.

Damit erlaubt es die vorliegende Erfindung, einen Gipsabdruck zu fertigen oder ein Stumpfmodell zu erhalten als Basis für einen Schaft, welcher dem Träger vor allem im Gehen und Stehen bequem passt. Dabei ist weniger handwerkliches Geschick als bisher erforderlich.

Somit kann die vorliegende Erfindung das Herstellen von Schäften für Prothesen der oberen und unteren Extremitäten des Menschen auf eine objektive Art, basierend auf direkt gewonnenen Messdaten ermöglichen. Die Erfindung ermöglicht somit die Herstellung eines gut angepassten Prothesenschafts, wobei jedoch die rein subjektiven und manuell ausgeführten kostenintensiven Tätigkeiten, die bei den Verfahren des Stands der Technik nötig sind, vermieden werden können.

Mittels der Erfindung ist es bei während des Anfertigens des Gipsabdrucks stehendem und dabei den Stumpf realitätsnah belastendem Patienten vorteilhaft auch erstmals möglich, bereits beim Anfertigen des Gipsabdrucks oder beim Vermessen eine Rückmeldung des Patienten über drückende oder schmerzende Stellen zu erhalten. Die spätere Tragesituation des Schafts wird vom Patienten bei Verwenden der erfindungsgemäßen Vorrichtung im Stehen sozusagen bereits während des Erstellens des Gipsabdrucks oder während der Vermessung "voraus"empfunden; unbefriedigend passende Abschnitte des späteren Schafts werden so frühzeitig erkannt bzw. antizipiert. Änderungswünsche oder Polsterungswünsche können so bereits beim Fertigen des Gipsabdrucks beim Vermessen vom Patienten angemeldet und vom Orthopädietechniker vorbereitet werden. Dies kann über die Zeitspanne, die bis zum Vorliegen des passenden, finalen Schafts vergeht, erheblich Arbeit und Zeit einsparen (helfen).

Da der Patient die Vorrichtung stehend verwenden kann und dabei die Membran mit Anteilen seines Körpergewichts belastet, bedarf es keiner Vorkehrung, um den Körpergliedstumpf davor zu bewahren, durch axiale Reaktionskräfte, welche durch die Druckkammer auf den Körpergliedstumpf wirken und diesen aus dem Inneren des Druckbehälters verdrängen könnten, verschoben zu werden. Der Patient muss mit anderen Worten, nicht in Stellung - bezogen auf den Druckbehälter - gehalten werden. Sein Körpergewicht kann dies übernehmen.

Das Anbinden des Verbinders an einen proximalen oder oberen Bereich der Membran vermeidet vorteilhaft radialen Druck auf die Membran in hierzu weiter distal gelegenen Abschnitten. Dies erlaubt es, Druck, welcher eine radiale Kompression des in der Membran steckenden Körpergliedstumpfs bewirken könnte, in einem - verglichen mit weiter proximal gelegenen Abschnitten - anatomisch schwächeren Abschnitt des Körpergliedstumpfs zu vermeiden. Als Ergebnis hiervon lässt sich eine wünschenswertere Geometrie des erzeugten Gipsabdrucks vor allem im distalen Stumpfabschnitt erzielen. Das Anbinden des Verbinders an einen proximalen Abschnitt der Membran vermeidet solche Verformungen des distalen Teils des Körpergliedstumpfs, was vorteilhaft zu einer korrekten Geometrie des Gipsabdrucks führen kann.

Die Erfindung ermöglicht gegenüber den herkömmlichen Vorrichtungen des Stands der Technik, auf eine objektive Art und Weise Schäfte für Prothesen zu erstellen. Dies gewährleistet eine bessere Versorgung durch verbesserte Passform und kann die Herstellungskosten dadurch senken, dass es keiner oder nur geringer kostenintensiver manueller Nacharbeitung und Anpassung bedarf. Zudem kann die Patientenversorgung beschleunigt werden, da die auch zeitaufwendigen Anpassungsschritte zumindest anzahlmäßig stark verringert werden können oder sogar ganz entfallen.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1: zeigt eine längs geschnittene erfindungsgemäße Vorrichtung in einer ersten Ausführungsform von der Seite;
- Fig. 2a, b: zeigen die erfindungsgemäße Vorrichtung aus Fig. 1 in verschiedenen Ausführungsformen in einer weiteren Schnittebene; und
- Fig. 3: zeigt eine längs geschnittene erfindungsgemäße Vorrichtung in einer weiteren Ausführungsform von der Seite.

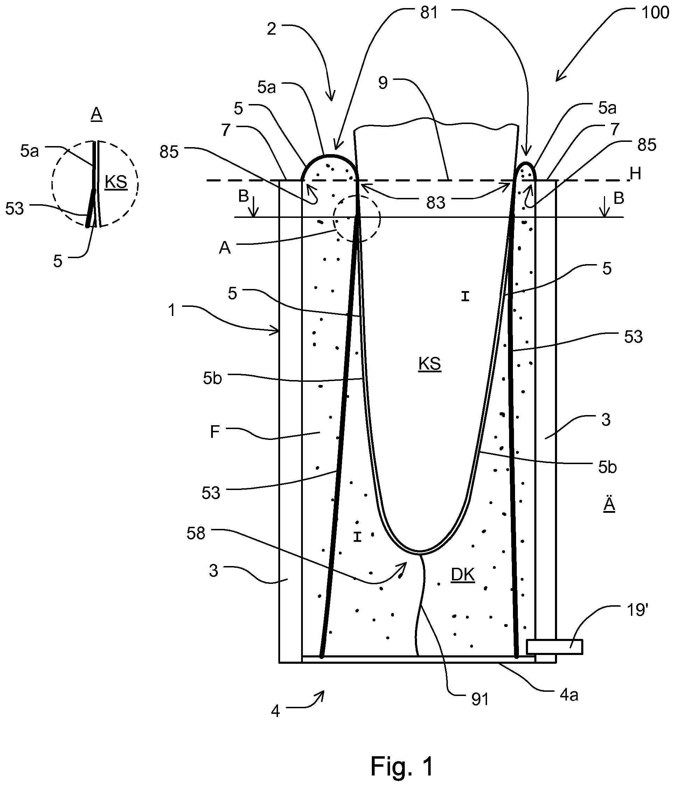

**Fig. 1** zeigt eine längs oder in Längsrichtung (also bezogen auf Fig. 1 von oben nach unten) geschnittene Vorrichtung 100 einer ersten erfindungsgemäßen Ausführungsform. Wenn hierin von "distal" die Rede ist, so ist hierunter eine Position oder Richtung nach unten zu verstehen. "Proximal" liegt dementsprechend oben, höher oder in Richtung dorthin.

Die erste Ausführungsform zeigt einen Druckbehälter 1 mit einer Druckkammer DK, einer Wandung 3 und einer Membran 5. Die Membran 5 weist einen ersten oder proximalen Membranabschnitt 5a auf, der mit einem oberen Rand 7 der Wandung 3 verbunden ist. Der erste Membranabschnitt 5a ist in diesem Ausführungsbeispiel optional verstärkt ausgeführt, was durch eine dickere Wandstärke (in Fig. 1 durch eine dickere Strichstärke dargestellt) gezeigt ist. Eine verstärkte Ausführung kann alternativ oder ergänzend mittels einer anderen Materialwahl, eines faserverstärkten Verbundmaterials, einer Materialbeschichtung oder in anderer Weise ausgeführt werden.

Weiterhin weist die Membran 5 einen zweiten oder distalen Membranabschnitt 5b auf, der im mittleren Abschnitt der Membran 5 angeordnet und mit dem ersten Membranabschnitt 5a verbunden oder einstückig mit diesem ist. Der zweite Membranabschnitt 5b ist optional nicht verstärkt, was durch eine geringere Wandstärke (in Fig. 1 durch eine dünnere Strichstärke angedeutet) dargestellt ist.

Die beiden unterschiedlichen Membranabschnitte 5a, 5b dienen unterschiedlichen Belastungssituationen der Membran 5. Bei einem Einführen und Ausführen eines Körpergliedstumpfs KS in die Membran 5, und damit in den Druckbehälter 1, wird insbesondere der erste Membranabschnitt 5a durch Reibkräfte zwischen dem Körpergliedstumpf KS und der Membran 5 belastet. Aufgrund dieser erhöhten Belastung ist der erste Membranabschnitt 5a optional verstärkt ausgeführt. Der zweite Membranabschnitt 5b hat insbesondere eine Dichtfunktion und Haltefunktion des Körpergliedstumpfs KS im Druckbehälter DK inne mit einer geringeren Belastung verglichen mit dem ersten Membranabschnitt 5a.

Zwei Verbinder 53, deren Funktion weiter unten näher beschrieben wird, sind in diesem Ausführungsbeispiel optional an der Verbindungsstelle oder Schnittstelle zwischen dem ersten 5a und zweiten 5b Membranabschnitt bzw. im Übergangsbereich zwischen beiden angeordnet. Insbesondere sind die Verbinder 53 mit dem ersten Membranabschnitt 5a (optional alternativ an einer anderen Stelle als der in Fig. 1 gezeigten Position) verbunden.

Die Verbindung zwischen Verbindern 53 und Membranabschnitt 5a oder 5b ist mit dem Bezugszeichen "A" bezeichnet. Im Beispiel der Fig. 1 liegt die Verbindung A um die Distanz B unterhalb der Höhe H und/oder dem oberen Rand der Wandung 3.

Die Distanz B kann optional zwischen 0 cm und 40 cm, bevorzugt zwischen 5 und 30 cm liegen, besonders bevorzugt zwischen 8 cm und 10 cm betragen.

Der Bereich der Verbindung A ist links in Fig. 1 vergrößert dargestellt.

Die Membran 5 ist mittels der wenigstens zwei Verbinder 53, die sich von einem proximalen Abschnitt der Membran 5 von letzterer erstrecken, an der Bodenfläche 4a, alternativ an einem Bereich der Wandung 3 (etwa in deren oberem, mittlerem oder unterem Bereich), vorzugsweise lösbar, befestigt.

Der proximale Bereich der Membran 5 ist hier exemplarisch als das obere Drittel der Membran 5 oder als ein Bereich im oberen Drittel der Membran 5 zu verstehen.

Der proximale Bereich der Membran 5 ist erkennbar der Einführöffnung 9 zugewandt oder liegt in deren Nähe.

Der Bereich, mit welchem die Membran 5 mit dem Verbinder 53 verbunden ist, liegt erkennbar nicht in einem zentralen, distalen Abschnitt 58 der Membran. Dies vermeidet vorteilhaft radialen Druck auf die Membran 5 in einem distalen Abschnitt bedingt des ein Auftreiben des Körpergliedstumpfs KS verhindernden Verbinders. Dieser Druck könnte eine radiale Kompression des in der Membran 5 steckenden Körpergliedstumpfs KS bewirken, was zu einer unerwünschten Geometrie des erzeugten Gipsabdrucks vor allem im distalen Stumpfabschnitt führen kann. Das Vorsehen des Verbinders 53 in einem proximalen Abschnitt kann beitragen, solche Verformungen des distalen Teils des Körpergliedstumpfs zu vermeiden, was vorteilhaft zu einer korrekten Geometrie des Gipsabdrucks führen kann. Zwar bewirkt der Verbinder 53 einen radialen Druck auf den Körpergliedstumpf KS in einem proximalen Abschnitt, bedingt durch seine proximale Anbindung an der Membran. Dort (also proximal oder, bezogen auf Fig. 1, "oben") weist der Körpergliedstumpf KS jedoch eine kräftigere Anatomie auf und ist weniger anfällig für die mittels der vorliegenden Erfindung in manchen Ausführungsformen vermeidbare radiale Kompression.

Der Druckbehälter 1, welcher rein optional, wie in Fig. 1 gezeigt, zylindrisch ist, weist optional eine erste Stirnseite 2 (oben in Fig. 1) und eine zweite Stirnseite 4 (unten in Fig. 1) auf. Die zweite Stirnseite 4 ist in der exemplarischen Ausführungsform der Fig. 1 mit einer Bodenplatte oder Bodenfläche 4a fluiddicht gegenüber einem Äußeren Ä verschlossen. Die Bodenfläche 4a kann aus demselben Material wie die Wandung 3 gefertigt sein. Sie kann einstückig mit dieser gefertigt sein.

Die Membran 5 trennt die Fluidkammer oder Druckkammer DK des Druckbehälters 1 fluiddicht gegenüber einem Äußeren der Fluidkammer oder Druckkammer DK, oder beispielsweise gegenüber dem Äußeren Ä, also einer Umgebung des Druckbehälters 1, oder, wie in Fig. 1 gezeigt, gegenüber einem in die Membran 5 eingeführten oder von dieser umgebenen Körpergliedstumpf KS.

Die Membran 5 kann an einem oberen, oftmals ringförmigen, rechteckigen, quadratischen oder auf andere Weise umlaufenden Rand 7 der Wandung 3, oder an anderer Stelle, mit dem Druckbehälter 1 fluiddicht verbunden sein.

Der obere Rand 7 liegt exemplarisch in einer Ebene, in welcher eine Einführöffnung 9 des Druckbehälters 1 liegt, oder begrenzt diese an deren Umfang. Die Einführöffnung 9 liegt optional in der mittels Strichlinie bezeichneten Ebene H.

Die Einführöffnung 9 dient dem Einführen des mit einer feuchten Gipsbinde umwickelten Körpergliedstumpfs KS in ein Inneres I des Druckbehälters 1.

Das Innere I ist der von der Wandung 3 begrenzte Rauminhalt des Druckbehälters 1. Es reicht von der mit der Bodenfläche 4a fluiddicht verschlossenen, zweiten Stirnseite 4 bis zur mit 9 bezeichneten, mittels Strichlinie angedeuteten Einführöffnung.

Die Druckkammer DK ist mit einem Fluid befüllt, hier exemplarisch mit einer durch Punkte angedeuteten Flüssigkeit F. Eine Befüllung mit Gas ist ebenfalls von der vorliegenden Erfindung erfasst.

In Fig. 1 ist die Vorrichtung 100 in einem Zustand gezeigt, in welchem der äußerst schematisch angedeutete Körpergliedstumpf KS des stehenden Patienten in das Innere I derart eingeführt ist, dass er zumindest in seinem distalen Abschnitt von der Membran 5 umgeben ist. Die Membran 5 liegt an der Gipsbinde über dem Körpergliedstumpf KS wie eine zweite Haut an, wobei zwischen Gipsbinde und Membran 5 weitere Schichten wie Liner oder dergleichen vorgesehen sein können.

Der Körpergliedstumpf KS ist im Beispiel der Fig. 1 vom stehenden Patienten mit dessen vollem Körpergewicht belastet. Die Menge der Flüssigkeit F ist in Kenntnis des Volumens des Inneren I oder des Druckbehälters 1 derart bemessen, dass der Körpergliedstumpf KS wenigstens derart tief durch die Einführöffnung 9 hindurch in den Druckbehälter 1 eintreten kann, dass die gesamte Fläche der Gipsbinde, jedenfalls aber soweit für den Abdruck relevant, mit der Membran 5 in Kontakt steht. Zugleich ist die Menge an Flüssigkeit F so bemessen, dass das distale Ende des Körpergliedstumpfs KS (unten in Fig. 1) den Boden des Druckbehälters 1 nicht berührt bzw. sich nicht hierauf abstützt. So ist sichergestellt, dass es der Druck des Fluids ist, auf welchem der Patient mit der eingeführten Extremität ruht, und dass die Gipsbinde mittels der Membran 5 an jeder Stelle denselben Druck erfährt.

Es ist Fig. 1 entnehmbar, dass die Membran 5 dann, wenn kein Körpergliedstumpf KS in den Druckbehälter 1 eingeführt ist, durch den Druck des Fluids, hier der Flüssigkeit, aufschwimmt oder -treibt und sich ein in Fig. 1 nicht gezeigter Flüssigkeitsspiegel einstellt. Die Form der Membran 5, die in Fig. 1 gezeigt ist, stellt also jene Form dar, welche die Membran 5 bei Belastung annimmt, wenn sie an dem eingeführten Körpergliedstumpf KS anliegt und durch diesen - im Beispiel der Fig. 1 - unter elastischer Dehnung in die Tiefe des Inneren I in Richtung auf die Bodenfläche 4a hin "mitgenommen" wird.

Es ist ferner Fig. 1 entnehmbar, dass die Wandung 3 und die Membran 5 dadurch, dass sie einen Fluidaustausch zwischen der Druckkammer DK und dem Äußeren Ä oder ein Austreten von Fluid aus der Druckkammer DK verhindern, ermöglichen, dass sich der gewünschte Druck innerhalb der Druckkammer DK des Druckbehälters 1 aufbaut, nicht aber hieraus entweichen oder sich abbauen kann.

Wie Fig. 1 zu entnehmen ist wird die Druckkammer DK optional somit von der Membran 5 und wenigstens Teilen der Wandung 3 gebildet, zu welchen hier auch die Bodenfläche 4a der Stirnseite 4 zählt.

In anderen als der in Fig. 1 gezeigten beispielhaften Ausgestaltung der vorliegenden Erfindung kann die Druckkammer DK aus einer rundum geschlossenen Membran, die ähnlich einem Ballon oder einer Blase im Inneren I des Druckbehälters 1 liegen kann, bestehen oder eine solche umfassen.

Weiterhin zeigt Fig. 1 einen optionalen Auslass 19' in der Seitenwand oder Wandung 3 des Druckbehälters 1. Dieser könnte, sofern vorhanden, auch an anderer Stelle des Druckbehälters 1 vorgesehen sein, z. B. in dessen Bodenfläche.

Der Auslass 19' verbindet das Innere I im Bereich der Druckkammer DK mit dem Äußeren Ä des Druckbehälters 1. Er erlaubt es, den Füllstand und/oder Druck innerhalb der Druckkammer DK gezielt zu verändern, beispielsweise indem Fluid aus der Druckkammer DK über den Auslass 19' abgelassen oder durch diesen eingelassen wird. Der Auslass 19' ist hierzu mit einem nicht näher dargestellten, fluiddicht schließbarem Ventil oder Sperrhahn versehen. Ungeachtet seiner Bezeichnung als Auslass kann dieser auch zum Einlassen von Fluid und damit zum Befüllen der Druckkammer DK verwendet werden.

Die Membran 5 ist in der Ausführungsform der Fig. 1 mittels der Verbinder 53 oder des Verbinders 53 rein exemplarisch mit der Bodenfläche 4a verbunden. Der Verbinder 53 ist optional nicht (oder nur in geringem Maße) elastisch oder dehnbar. Die Membran 5 ist optional in Längsrichtung ebenfalls nicht (oder nur in geringem Maße) elastisch oder dehnbar.

Der Verbinder 53 erlaubt optional ein Auftreiben der Membran 5 nach proximal (nach oben in Fig. 1) in geringem und/oder vorbestimmtem Umfang. Daher stellt sich an der zweiten Stirnseite 2 eine mehr oder weniger ringförmige, jedenfalls aber in ihrem Umfang geschlossene Auftreibung 81 ein. Die Auftreibung 81 ist bedingt durch die Schnittdarstellung der Fig. 1 als Erhöhungen links und rechts des Körpergliedstumpfs KS zu sehen. Der Verbinder 53 kann vorgesehen sein, um eine solche Auftreibung zu verhindern.

Die Membran 5 liegt dem Körpergliedstumpf KS (oder einer über diesen gezogenen Gipsbinde, einem Liner oder dergleichen) in einer mehr oder weniger ringförmigen, jedenfalls aber in ihrem Umfang geschlossenen Kontaktpunktfläche 83 an. Die Kontaktpunktfläche 83 ist die im Umfang geschlossene Linie oder Fläche, welche die Punkte umfasst, an welchen der Körpergliedstumpf KS nach proximal letztmalig oder abschließend Kontakt mit der Membran 5 hat. Die Kontaktpunktfläche könnte auch als Kontaktfläche oder -linie bezeichnet werden.

Gleichzeitig liegt die Membran 5 an ihrem radialen Umfang im Bereich der zweiten Stirnseite 2 der Wandung 3 in einem in seinem Umfang ebenfalls geschlossenen Übergangsabschnitt 85 an oder ist hiermit befestigt. Der Übergangsabschnitt 85 kann als Bereich verstanden werden, in welchem die Begrenzung der Druckkammer DK von einer Begrenzung durch die Wandung 3 in eine Begrenzung durch die Membran 5 übergeht. Dies ist in Fig. 1 gut dadurch zu erkennen, dass die Druckkammer DK unterhalb der Pfeilspitze der Bezugszeichenlinie zum Bezugszeichen 85 allein durch die Wandung 3 begrenzt wird, oberhalb allein durch die Membran 5, die sich ihrerseits nicht gegen einen Abschnitt der Wandung 3 abstützen kann.

Die Befestigung der Membran 5 im Übergangsabschnitt 85 kann auf vielfältige Weise erfolgen. Sie erfolgt in dieser wie in beliebigen anderen Ausführungsformen jedoch nicht mittels des Verbinders 53. Die Verbindung der Membran 5 mittels des Verbinders 53 erfolgt im Bereich der Verbindung A.

Die Auftreibung 81 kann durch Ablassen von Fluid oder Einführen von Fluid mittels des Auslasses oder Einlasses 19' im Zusammenwirken mit dem Verbinder 53 so eingestellt werden, dass sich die Kontaktpunktfläche 83 und der Übergangsabschnitt 85 in ein und derselben Höhe H (H meint die Höhe über der Bodenfläche 4a), angedeutet durch die Strichlinie in Fig. 1, befinden. Ist dies geschehen, so liegen nach Erfahrung des Erfinders der vorliegenden Erfindung optimale Druckverhältnisse zum Fertigen des Gipsabdrucks vor.

Selbst wenn der Verbinder 53 nicht in seiner Länge verstellbar ist, stellt er vorteilhaft sicher, dass mehr als ein vorbestimmtes Maß an Auftreibung 81 über die Höhe H hinaus nicht eintreten kann. Ein vorbestimmtes Maß kann 1 bis 4 cm betragen, besonders bevorzugt sind rund 2 cm.

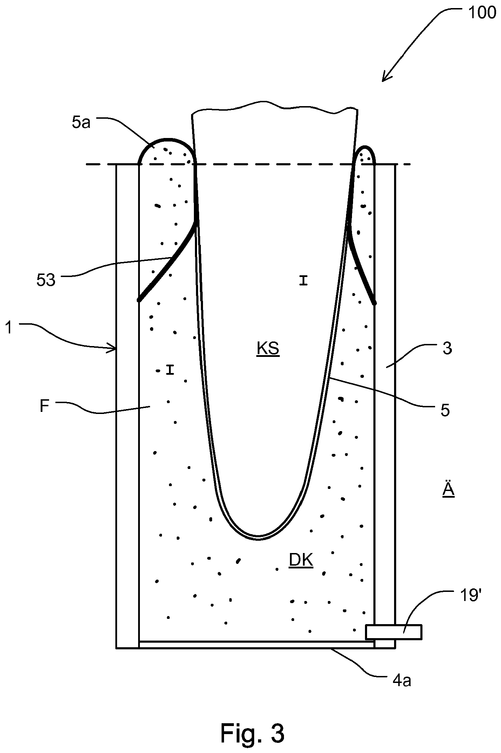

Fig. 1 zeigt einen distalen Verbinder 91, welcher - in jeder beliebigen Ausführungsform - optional vorgesehen sein kann, obgleich er z. B. in Fig. 3 nicht gezeigt ist.

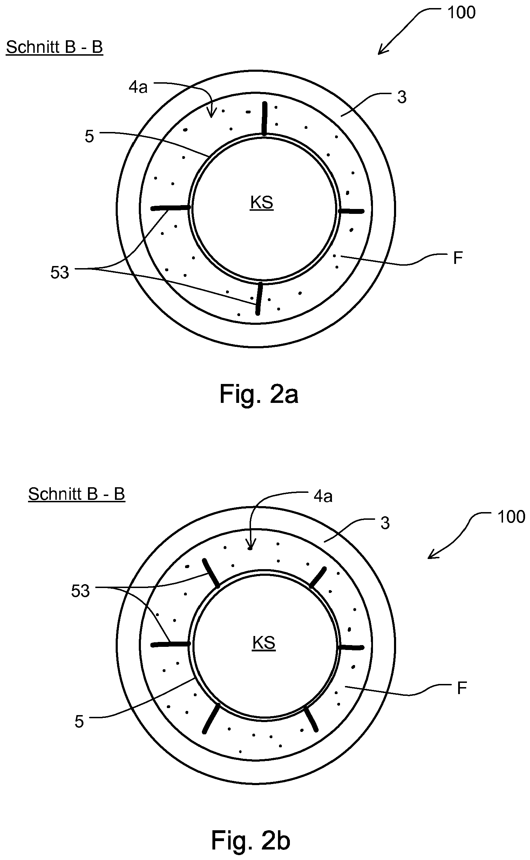

**Fig. 2a** zeigt die erfindungsgemäße Vorrichtung 100 der Fig. 1 in einer weiteren Schnittebene B-B. Die Schnittebene B-B zeigt die Vorrichtung 100 im Querschnitt an einer Position in Längsrichtung, in der die Verbinder 53 mit der Membran 5 verbunden sind. Die Blickrichtung der Schnittebene B-B ist in Fig. 2a nach unten auf die Bodenfläche 4a gerichtet. In Fig. 2a sind vier Verbinder 53, jeweils um ca. 90 Grad in Umfangsrichtung versetzt, angeordnet. Alternativ könnten die Verbinder unregelmäßig über dem Umfang angeordnet sein. Sie können zudem in beliebiger Anzahl und unter beliebigem Abstand zueinander vorliegen (etwa alle 5 cm entlang des Umfangs).

Die Verbinder 53 sind an ihrem einen Ende an der Membran 5, und an ihrem anderen Ende an der Bodenfläche 4a befestigt. Inmitten der Membran 5 ist der Körpergliedstumpf KS angeordnet.

Die Verbinder 53 können miteinander verbunden sein, etwa indem sie, z. B. alle, in eine ringförmigen Einrichtung einstrahlen oder mit einer ringförmigen Einrichtung verbunden sind, die jeweils um den Umfang der Membran 5 geführt ist.

**Fig. 2b** zeigt die Anordnung aus Fig. 2a, jedoch mit sechs Verbindern 53, die jeweils um ca. 60 Grad in Umfangsrichtung versetzt angeordnet sind. Alternativ könnten die Verbinder unregelmäßig über dem Umfang angeordnet sein.

**Fig. 3** zeigt die längs geschnittene erfindungsgemäße Vorrichtung 100 in einer weiteren Ausführungsform von der Seite. In dieser Ausführungsform sind die Verbinder 53 an der Wandung 3 des Druckbehälters 1 befestigt. Funktional haben die an der Wandung 3 befestigten Verbinder 53 die gleiche Aufgabe, nämlich ein ungewünschtes Auftreiben der Membran 5 zu verhindern. Eine Befestigung an der Wandung 3 kann Vorteile hinsichtlich der Montage und/oder Handhabung während des Einsatzes der erfindungsgemäßen Vorrichtung 100 haben.

### Bezugszeichenliste

- 100: Vorrichtung
- 1: Druckbehälter
- 2: erste Stirnseite
- 3: Wandung
- 4: zweite Stirnseite, kann optional mit der Bodenfläche verschlossen sein
- 4a: Bodenfläche
- 5: Membran
- 7: oberer Rand
- 5a: proximaler oder erster Membranabschnitt; verstärkter Abschnitt der Membran
- 5b: distaler oder zweiter Membranabschnitt
- 9: Einführöffnung
- 19': Auslass, Einlass
- 53: Verbinder
- 58: zentraler oder mittlerer Bereich oder Abschnitt der Membran
- 81: Auftreibung der Membran
- 83: Kontaktpunktfläche
- 85: Übergangsabschnitt
- 91: distaler Verbinder

- A: Verbindung
- Ä: Äußeres des Druckbehälters
- B: Distanz
- DK: Druckkammer des Druckbehälters
- I: Inneres des Druckbehälters
- F: Fluid oder Flüssigkeit
- H: Höhe der Druckkammer
- KS: Körpergliedstumf

## Patentansprüche

1. Medizinische Vorrichtung (100) zur Verwendung bei der Anfertigung eines Gipsabdrucks oder eines Datenmodells eines Körpergliedstumpfs, insbesondere eines Unterschenkelstumpfs, wobei die Vorrichtung (100) wenigstens aufweist:
- einen Druckbehälter (1) mit einer im Gebrauch der Vorrichtung oberen Stirnseite als erste Stirnseite (2) und einer im Gebrauch unteren Stirnseite als zweite Stirnseite (4), mit einer Fluidkammer oder einer Druckkammer (DK) zur Aufnahme oder Speicherung eines unter Druck stehenden Fluids (F), wobei die Fluidkammer oder Druckkammer (DK) ein abgeschlossener Raum ist, in welchem das Fluid einem Druck oberhalb des Atmosphärendrucks (kurz: Atmosphäre) ausgesetzt werden kann, wobei der Druckbehälter (1) eine Wandung (3) aus einem ersten Material aufweist, wobei die Wandung (3) ein Inneres (I) des Druckbehälters (1) gegenüber einem Äußeren (Ä) begrenzt, wobei der Druckbehälter (1) in der ersten Stirnseite (2) eine Einführöffnung (9) zum Einführen des Körpergliedstumpfs (KS) in das Innere (I) des Druckbehälters (1) aufweist; und
- eine fluiddichte Membran (5) aus wenigstens einem zweiten Material, welche angeordnet ist, um die Fluidkammer oder die Druckkammer (DK)zu begrenzen,
- wobei wenigstens ein Abschnitt der Membran (5) mittels wenigstens eines Verbinders (53) an dem Druckbehälter (1) befestigt ist,
**dadurch gekennzeichnet, dass**
der Abschnitt in einem proximalen oder der Einführöffnung (9) zugewandten Bereich der Membran (5) liegt, wobei
der wenigstens eine Verbinder (53) an einer Bodenfläche (4a), an einer Seitenfläche der Wandung (3) oder an der zweiten Stirnseite (4) des Druckbehälters (1) befestigt ist.

2. Medizinische Vorrichtung (100) nach Anspruch 1, wobei der wenigstens eine Verbinder (53) mit einem verstärkten Abschnitt (5a) der Membran (5) verbunden ist.

3. Medizinische Vorrichtung (100) nach dem vorangegangenen Anspruch, wobei der verstärkte Abschnitt (5a) der Membran (5) einerseits mit dem wenigstens einen Verbinder (53) und andererseits mit der Wandung (3) des Druckbehälters (1) verbunden ist.

4. Medizinische Vorrichtung (100) nach dem vorangegangenen Anspruch, wobei wenigstens zwei Verbinder (53) an einem Umfang der Membran (5, 5a) befestigt sind.

5. Medizinische Vorrichtung (100) nach einem der vorangegangenen Ansprüche, mit:
- einem Auslass (19), welcher eine Fluidverbindung zwischen der Fluidkammer oder Druckkammer (DK) und dem Äußeren (Ä) des Druckbehälters (1) ist; und
- einem Ventil, einer Sperreinrichtung oder einem Sperrhahn, zum Öffnen und Schließen des Auslasses (19) oder der Fluidverbindung.

6. Medizinische Vorrichtung (100) nach einem der Ansprüche 3 oder 4, wobei der Verbinder (53) nicht elastisch und/oder nicht dehnbar ist.

7. Medizinische Vorrichtung (100) nach einem der vorangegangenen Ansprüche, wobei die Membran (5) aus einem Material gefertigt ist oder ein solches aufweist, welches in einer ersten Richtung des Materials eine andere Elastizität oder Dehnbarkeit als in einer zweiten, zur ersten senkrecht stehenden, Richtung aufweist.

8. Medizinische Vorrichtung (100) nach einem der vorangegangenen Ansprüche, wobei die Membran (5) aus einem Material gefertigt ist oder ein solches aufweist, welches in einer ersten Richtung und/oder in einer zweiten Richtung des Materials Fasern (55a, 57a; 55b, 57b) aufweist, welche vorzugsweise in eine Matrix eingebettet oder vorzugsweise mit dieser auf andere Weise verbunden sind.

9. Medizinische Vorrichtung (100) nach einem der vorangegangenen Ansprüche, wobei die Membran (5) in einer ersten Richtung hiervon und/oder in einer zweiten Richtung hiervon nicht dehnbar oder nicht elastisch ist.

## Claims

1. A medical apparatus (100) for use in the production of a plaster cast or a data model of a limb stump, in particular a lower leg stump, the apparatus (100) comprising at least:
- a pressure vessel (1) having, when the apparatus is in use, an upper front side as a first front face (2) and a lower front face as a second front face (4), with a fluid chamber or a pressure chamber (DK) for receiving or storing a fluid (F) under pressure, wherein the fluid chamber or pressure chamber (DK) is a closed space in which the fluid can be exposed to a pressure above the atmospheric pressure (in short: atmosphere), wherein the pressure vessel (1) comprises a wall (3) made of a first material,
the wall (3) delimiting an interior (I) of the pressure vessel (1) against an exterior (Ä), wherein the pressure vessel (1) comprises an insertion opening (9) in the first front face (2) for inserting the limb stump (KS) into the interior (I) of the pressure vessel (1); and
- a fluid-tight membrane (5) made of at least one second material, which is arranged to delimit the fluid chamber or the pressure chamber (DK);
- wherein at least one section of the membrane (5) is fastened to the pressure vessel (1) by means of at least one connector (53),
**characterized in that**:
the section lies in a region of the membrane (5) being proximal or facing the insertion opening (9), wherein
the at least one connector (53) is fastened to a bottom surface (4a), to a side surface of the wall (3) or to the second front site (4) of the pressure vessel (1).

2. The medical apparatus (100) according to claim 1, wherein the at least one connector (53) is connected to a reinforced section (5a) of the membrane (5).

3. The medical apparatus (100) according to the preceding claim, wherein the reinforced section (5a) of the membrane (5) is connected on the one hand to the at least one connector (53) and on the other hand to the wall (3) of the pressure vessel (1).

4. The medical apparatus (100) according to the preceding claim, wherein at least two connectors (53) are fastened to a circumference of the membrane (5, 5a).

5. The medical apparatus (100) according to anyone of the preceding claims, having:
- an outlet (19) which is a fluid communication between the fluid chamber or the pressure chamber (DK) and the exterior (Ä) of the pressure vessel (1); and
- a valve, a locking device or a stopcock, for opening and closing the outlet (19) or the fluid communication.

6. The medical apparatus (100) according to anyone of claims 3 or 4, wherein the connector (53) is not elastic and/or not stretchable.

7. The medical apparatus (100) according to anyone of the preceding claims, wherein the membrane (5) is made of a material or comprises such a material having a different elasticity or stretchability in a first direction of the material than in a second direction perpendicular to the first one.

8. The medical apparatus (100) according to anyone of the preceding claims, wherein the membrane (5) is made of a material or comprises such a material having fibers (55a, 57a; 55b, 57b) in a first direction and/or in a second direction of the material, which are preferably embedded into a matrix or preferably connected to it in another way.

9. The medical apparatus (100) according to anyone of the preceding claims, wherein the membrane (5) is not stretchable or not elastic in a first direction thereof and/or in a second direction thereof.

## Revendications

1. Un appareil médical (100) destiné à être utilisé dans la fabrication d'un moulage en plâtre ou d'un modèle de données d'un moignon, notamment d'un moignon de bas de jambe, l'appareil (100) comprenant au moins:
- un réservoir à pression (1) ayant, lors de l'utilisation de l'appareil, une face frontale supérieure en tant que première face frontale (2) ainsi qu'une face frontale inférieure en tant que seconde face frontale (4), avec une chambre à fluide ou une chambre à pression (DK) pour recevoir ou stocker un fluide (F) sous pression, où la chambre à fluide ou la chambre à pression (DK) est un espace clos dans lequel le fluide peut être soumis à une pression supérieure à la pression atmosphérique (en bref: l'atmosphère), où le réservoir à pression (1) comprend une paroi (3) constituée d'un premier matériau, la paroi (3) délimitant un intérieur (I) du réservoir à pression (1) par rapport à un extérieur (Ä), où le réservoir à pression (1) comprend une ouverture d'insertion (9) dans la première face frontale (2) pour insérer le moignon (KS) à l'intérieur (I) du réservoir à pression (1); et
- une membrane (5) étanche aux fluides, constituée d'au moins un second matériau, qui est agencée de manière à délimiter la chambre à fluide ou la chambre à pression (DK);
- où au moins une partie de la membrane (5) est fixée au réservoir à pression (1) au moyen d'au moins un connecteur (53),
**caractérisé en ce que**:
la partie se trouve dans une zone de la membrane (5) étant proximale ou faisant face à l'ouverture d'insertion (9), où
au moins un connecteur (53) est fixé sur une surface de fond (4a), sur une surface latérale de la paroi (3) ou sur la seconde face frontale (4) du réservoir à pression (1).

2. L'appareil médical (100) selon la première revendication, où, au moins un connecteur (53) est relié à une partie renforcée (5a) de la membrane (5).

3. L'appareil médical (100) selon la revendication précédente, où la partie renforcée (5a) de la membrane (5) est reliée d'une part à au moins un connecteur (53) et d'autre part à la paroi (3) du réservoir à pression (1).

4. L'appareil médical (100) selon la revendication précédente, où au moins deux connecteurs (53) sont fixés à une périphérie de la membrane (5, 5a).

5. L'appareil médical (100) selon l'une quelconque des revendications précédentes, ayant:
- une sortie (19) qui est une communication fluidique entre la chambre à fluide ou la chambre à pression (DK) et l'extérieur (Ä) du réservoir à pression (1); et
- une soupape, un dispositif de verrouillage ou un robinet d'arrêt, pour l'ouverture et la fermeture de la sortie (19) ou de la communication fluidique.

6. L'appareil médical (100) selon l'une quelconque des revendications 3 à 4, où le connecteur (53) n'est pas élastique et/ou extensible.

7. L'appareil médical (100) selon l'une quelconque des revendications précédentes, où la membrane (5) est fabriquée à partir d'un matériau, ou comprend un tel matériau, ayant une élasticité ou extensibilité différente dans une première direction du matériau et dans une seconde direction perpendiculaire à la première.

8. L'appareil médical (100) selon l'une quelconque des revendications précédentes, où la membrane (5) est fabriquée à partir d'un matériau, ou comprend un tel matériau, ayant des fibres (55a, 57a; 55b, 57b) dans une première direction et/ou dans une seconde direction du matériau, lesquelles sont de préférence incorporées dans une matrice ou de préférence reliées à celle-ci d'une autre manière.

9. L'appareil médical (100) selon l'une quelconque des revendications précédentes, où la membrane (5) n'est pas extensible ou élastique dans une première direction de celle-ci et/ou dans une seconde direction de celle-ci.
